Europäisches Patentamt

European Patent Office

Office européen des brevets

(19)

(11) Veröffentlichungsnummer: **0 209 121 B1**

# EUROPÄISCHE PATENTSCHRIFT

(12)

(45) Veröffentlichungstag der Patentschrift: **29.01.92**

(21) Anmeldenummer: **86109726.9**

(22) Anmeldetag: **16.07.86**

(51) Int. Cl.⁵: **A61K 9/22**, A61K 9/70, A61L 15/16

(54) **Pharmazeutische Zubereitung mit kontrollierter und verzögerter Wirkstofffreigabe und Verfahren zu deren Herstellung.**

(30) Priorität: **19.07.85 DE 3525767**

(43) Veröffentlichungstag der Anmeldung:
**21.01.87 Patentblatt 87/04**

(45) Bekanntmachung des Hinweises auf die Patenterteilung:
**29.01.92 Patentblatt 92/05**

(84) Benannte Vertragsstaaten:
**AT BE CH DE FR GB IT LI LU NL SE**

(56) Entgegenhaltungen:
**EP-A- 0 086 997**
**EP-A- 0 122 574**
**FR-A- 2 497 457**
**US-E- 28 316**

(73) Patentinhaber: **BOEHRINGER INGELHEIM KG**
**Postfach 200**
**W-6507 Ingelheim am Rhein(DE)**

(84) Benannte Vertragsstaaten:
**BE CH DE FR IT LI LU NL SE AT**

Patentinhaber: **BOEHRINGER INGELHEIM IN-**
**TERNATIONAL GmbH**
**Postfach 20**
**W-6507 Ingelheim am Rhein(DE)**

(84) Benannte Vertragsstaaten:
**GB**

(72) Erfinder: **Zierenberg, Bernd, Dr.**
**Goethestrasse 1**
**W-6530 Bingen/Rhein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 209 121 B1

**Beschreibung**

Die Erfindung betrifft eine pharmazeutische Zubereitung in Form eines Trägermaterials mit einem Arzneimittelwirkstoff mit kontrollierter und verzögerter Wirkstofffreigabe und verfahren zu deren Herstellung.

Es ist bekannt, pharmazeutische Zubereitungen in Form von Kapseln, Tabletten und Filmen herzustellen, die eine gleichbleibende Wirkstofffreisetzung gewährleisten und somit eine konstante Konzentration des Wirkstoffs im Körper aufrechterhalten. Bei Kapseln und Tabletten wird üblicherweise die Freigabe des Wirkstoffs durch geeignete Überzüge, wie beispielsweise bei Filmtabletten, gesteuert. Depotformen können auch aus Granulatgemischen unterschiedlicher Trägerstoffe hergestellt werden, die den Arzneimittelwirkstoff verschieden schnell freisetzen. Eine teilbare Tablette mit verzögerter Wirkstofffreigabe ist aus der DE-OS 33 14 003 bekannt. Bei dieser aus einem emulsionspolymerisiertem Polyacrylat hergestellten Tablette wird die Freigaberate über die Korngröße wie auch Korngrößenverteilung gesteuert.

Bei Systemen zur transdermalen Applikation kann die Freigabe des Wirkstoffs durch besondere Membrane (US-PS 3 731 683) oder aber auch mit Hilfe von Adjuvantien, beispielsweise Amine, Fette oder höhere Alkohole, eingestellt werden.

Die aus dem Stand der Technik bekannten pharmazeutischen Zubereitungen mit einer verzögerten Wirkstofffreigabe erwiesen sich in der Praxis nicht immer als voll befriedigend. Einerseits ändern Tabletten, die mit einem Film überzogen sind, nach der Teilung ihr Freigabeverhalten, andererseits ist der aus der DE-OS 33 14 003 bekannten Kontrollmöglichkeit der Wirkstofffreigabe durch die Einstellung der Korngröße Grenzen gesetzt, da die Korngröße nicht beliebig frei wählbar ist. Aufgrund technischer Probleme, wie z.B. Inhomogenität, Mindestanzahl der mit Wirkstoff beladenen Teilchen, ist die Korngröße sowohl nach oben wie auch nach unten begrenzt, so daß nicht in jedem Fall eine dem Wirkstoff entsprechende optimale Freigaberate eingestellt werden kann.

Überraschenderweise wurde gefunden, daß bei einer pharmazeutischen Zubereitung aus einem emulsionspolymerisierten Trägermaterial die Freigaberate durch einfache Variation der Teilchengröße der emulsionspolymerisierten Polymerkügelchen wie auch durch Variation der Glastemperatur des Polymeren eingestellt werden kann.

Durch die Kombination der Variation der Korngröße, der Teilchengröße und der Glastemperatur besteht bei pulverförmigen pharmazeutischen Zubereitungen, wie z.B. Tabletten oder Kapseln die Möglichkeit, die Wirkstofffreigabe der pharmazeutischen Zubereitung in einem weiten Bereich einzustellen. Bei pharmazeutischen Zubereitungen in Form von transdermalen Filmen kann die Freigaberate durch Variation der Schichtdicke im Bereich zwischen 40 und 200 $\mu$m, bevorzugt 60 bis 140 $\mu$m, der Teilchengröße und der Glastemperatur eingestellt werden.

Die Teilchengröße bezieht sich auf den Teilchendurchmesser des polymeren Materials nach dessen Herstellung und beträgt erfindungsgemäß 50 bis 500 nm. Die Teilchengröße (Durchmesser) kann in Abhängigkeit von den Polymerisationsbedingungen eingestellt werden. Eine Abnahme der Teilchengröße bewirkt eine Erhöhung der Freisetzungsrate.

Die Glastemperatur kann durch Änderung der Monomerzusammensetzung eingestellt werden und liegt erfindungsgemäß zwischen -20° und + 80°C, bevorzugt zwischen - 20° und + 40°C, besonders bevorzugt zwischen - 10° und + 30°C. Ein Anstieg der Glastemperatur ist mit einer Erniedrigung der Freisetzungsrate verbunden.

Die Korngröße liegt in einem Bereich zwischen 10 und 500 $\mu$m. Zur Einstellung der Korngröße wird das emulsionspolymerisierte Polymer mit einer definierten Teilchengröße zusammen mit dem Wirkstoff in einem geeigneten Lösungsmittel gelöst, anschließend zu einem Film vergossen und das Lösungsmittel verdampft. Der Film wird dann, gegebenenfalls unterhalb seiner Glastemperatur, auf die gewünschte Korngröße vermahlen und falls erforderlich gesiebt.

Als Trägermaterial sind solche Polymere geeignet, die sich nach dem Emulsionspolymerisationsverfahren herstellen lassen, wie z.B. PVC, Polylactide, Polystyrol, Polyvinylacetat, Polybutadien, Polyacrylnitril, Polyvinylester, Polyvinylether und deren Copolymere. Bevorzugt sind emulsionspolymerisierte Copolymerisate von Methyl- und/oder Ethylestern der Acryl- und Methacrylsäure. Das Molekulargewicht der Emulsionspolymere sollte zwischen $10^4$ und $10^7$ betragen. Die Gewinnung des Trägermaterials als Feststoff kann z.B. durch Gefriertrocknung erfolgen, hierbei bleiben die Polymerisatteilchen in ihrer Form und Größe erhalten.

Die Herstellung der erfindungsgemäßen pharmazeutischen Zubereitung kann in der Art erfolgen, daß man ein Emulsionspolymerisat mit der gewünschten Teilchengröße und Glastemperatur zusammen mit dem Wirkstoff in einem möglichst leichtflüchtigen Lösungsmittel suspendiert.

Als Lösungsmittel für die Wirkstoff und Emulsionspolymerisat enthaltene Suspension kommen solche Solventien in Frage, die einen niedrigen Verdampfungspunkt aufweisen, wie z.B. niedrig siedende Alkohole, wie z.B. Methanol, Ethanol, Aceton, Methylethylketon,halognierte Kohlenwasserstoffe,wie z.B. Methylenchlorid, Essigsäuremethyl- und ethylester, Acetessigsäuremethyl- und -ethylester, oder Ether, wie z.B. Tetrahydrofuran oder Dioxan oder Dimethylsulfoxid, niedrig siedende Frigene, bzw. auch Gemische dieser Lösungsmittel.

Die Freigaberate des Wirkstoffes kann durch Adjuvantien, d.h. diffusionsbeeinflussende Stoffe zusätzlich variiert werden. Zu diesen sogenannten Adjuvantien zählen z.B. Polyvinylpyrrolidon, Celluloseester, Amine, Fette oder höhere Alkohole. Wenn es gewünscht wird, können diese Stoffe der Wirkstofflösung zugesetzt werden.

Diese Lösung kann wie folgt weiterverarbeitet werden:

Zur Herstellung einer wirkstoffhaltigen pharmazeutischen Zubereitung in Form eines Films zur transdermalen therapeutischen Applikation wird die erhaltene Lösung zu einem Film definierter Schichtdicke ausgegossen, die so bemessen ist, daß der Film nach Verdunsten des Lösungsmittels eine Schichtdicke zwischen 40 und 200 $\mu$m, bevorzugt 60 bis 140 $\mu$m, aufweist. Anschließend kann der Film mit einer dampfundurchlässigen Rückschicht und gegebenenfalls einer Klebeschicht versehen und konfektioniert werden.

Zur Herstellung einer wirkstoffhaltigen pharmazeutischen Zubereitung in Pulverform wird das Lösungsmittel der oben beschriebenen Lösung, bei feuchtigkeits- oder oxidationsempfindlichen Arzneimitteln,gegebenenfalls unter Schutzgas, abgezogen und der erhaltene Rückstand unterhalb seiner Glastemperatur auf die gewünschte Korngröße zwischen 10 und 500 $\mu$m vermahlen. Wenn es erforderlich ist, kann die Vermahlung auch in Gegenwart inerter Hilfs- oder Trägerstoffe, wie z.B. Lactose, Magnesiumstearat, feinverteilte Kieselerde oder ähnliche feinteilige Gleitmittel erfolgen. Das Massenverhältnis von wirkstoffhaltigen Emulsionspolymerisat zu feinteiligem Füllstoff kann innerhalb weiter Grenzen variiert werden und liegt bei etwa 10 : 1 bis 1 : 4. Die Weiterverarbeitung zur Tabletten oder Kapseln erfolgt nach bekannten Verfahren mit üblichen Hilfsstoffen, wie z.B. Bindemittel, inerte Füllstoffe, Gleit- und Schmiermittel sowie Sprengmittel.

Eine bevorzugte Ausführungsform ist eine teilbare Tablette, da die erfindungsgemäße Zubereitung ihre vorteilhaften Eigenschaften auch in jedem Bruckstück beibehält. Bei der Herstellung von Filmen zur transdermalen Applikation kann bei vorgegebener Filmdicke und eingearbeiteter Arzneimittelmenge die Freigaberate durch Variationen der Teilchengröße und der Glastemperatur eingestellt werden. Es ist bekannt, daß bei einer erhöhten Beladung des Trägermaterials die Freisetzungsrate erheblich gesteigert wird, so daß eine verzögerte Freigabe des Wirkstoffs nicht mehr gewährleistet ist. Dies könnte durch eine Vergrößerung der Korngröße kompensiert werden, hierbei bestünde jedoch die Gefahr, daß die Mindestanzahl der beladenen Teilchen in einer Tablette unter die kritische Zahl von 400 sinkt (hierbei ist eine homogene Verteilung des Arzneimittelwirkstoffs von Tablette zu Tablette nicht mehr sichergestellt). Erfindungsgemäß kann das Problem durch eine höhere Glastemperatur des emulsionspolymerisierten Trägermaterials und eine größere Teilchengröße gelöst werden, die Korngröße kann dabei weitestgehend konstant gehalten werden. Dies ermöglicht bei Tabletten, Kapseln oder Filmen eine wesentlich höhere Beladung des Trägermaterials mit einem Arzneimittel.

Erfindungsgemäß können auch günstigere Verhältnisse von Arzneistoff zu Trägermaterial eingestellt und damit das Depotprinzip auf höher zu dosierende Arzneistoffe ausgeweitet werden, da die Freigaberate auch bei höherer Beladung durch die beschriebenen Parameter, Teilchengröße, Korngröße und Glastemperatur in einem weiten Bereich eingestellt werden kann.

In der Regel enthalten die erfindungsgemäßen Zubereitungen zwischen 0.1 und 30 Gew.-%, bevorzugt 1 bis 10 Gew.-% Wirkstoff.

Beispiele

A) Filme

Beispiel 1:

Es werden die in Tab. I aufgeführten emulsionspolymerisierten Polyacrylate zu transdermalen Filmen verarbeitet und auf ihre Freisetzungsraten hin untersucht.

Tab. I

| emulsionspolymerisiertes Polyacrylat | | |
|---|---|---|
| Teilchendurchmesser der Latexteilchen | Glastemperatur | Filmcharge |
| 50 - 100 nm | - 8°C | A |
| 50 - 100 nm | + 29°C | B |
| 100 - 150 nm | - 8°C | C |

Die Latexpartikel werden durch Abtrocknen des Wassers aus der jeweiligen Dispersion (Fa. Röhm GmbH, Darmstadt) gewonnen. 21,3 g des getrockneten Polyacrylates werden zusammen mit 1,6 g Clonidin in 142 g Aceton gelöst und auf einer ebenen Fläche zu Filmen ausgegossen. Die Schichtdicke betrug nach dem Abdampfen des Lösungsmittels 80 $\mu$m. Die Freisetzungsrate wurde gegenüber einem wäßrigen Medium von pH 7, bei 37°C, bestimmt, die Freigabeanalytik erfolgte mittels HPLC.

Die für die drei unterschiedlichen Latextypen ermittelten Diffusionskoeffizienten sind in der Tabelle II aufgeführt:

Tab. II

| Filmcharge | Diffusionskoeffizient D [cm$^2$/Tag] |
|---|---|
| A | 5.5 10$^{-6}$ |
| B | 4.4 10$^{-6}$ |
| C | 1.4 10$^{-6}$ |

In der Abb. I sind die Freigaberaten für die drei physikalisch unterschiedlichen Latices aufgeführt. Wie aus den Kurvenverläufen ersichtlich, kann das Freigabeverhalten sowohl durch die Teilchengröße wie auch die Glastemperatur der Latices beeinflußt werden.

Beispiel 2:

Es werden die in Tab. III aufgeführten emulsionspolymerisierten Copolymerisate aus Vinylchlorid und Acrylsäureester zu transdermalen Filmen verarbeitet und auf ihre Freisetzungsrate hin untersucht.

Tabelle III

| | VA 381A | VA 381 B |
|---|---|---|
| Feststoffgehalt an Polymeren in der Dispersion | 44,6 % | 44,7 % |
| Filmbilungstemperatur (~Glastemperatur) | ~67°C | ~0°C |
| Durchmesser der Latices (Teilchengröße) | 261 nm | 262 nm |

Die beide über die Emulsionspolymerisation erhaltenen Materialien unterscheiden sich in der Glastemperatur und damit in der Weichheit des Polymeren, die Latexgröße ist in beiden Produkten gleich. Das Polymer wird durch Abtrocknen der wäßrigen Dispersion erhalten, anschließend zusammen mit dem Arzneistoff Clonidin in Methylenchlorid gelöst und in der bekannten Weise zu Filmen von ca. 100 $\mu$m Schichtdicke ausgegossen. Die Freigabe an Arzneistoff aus dem CPA-Film * der Größe 3 cm$^2$ wird im USP-Tester ermittelt und die Gehaltsbestimmung über HPLC durchgeführt.

In Tabelle IV sind die zu verschiedenen Zeiten freigegebenen Mengen an Clonidin (in Prozent) aufgeführt. Die Freigaberate nimmt mit steigender Glastemperatur ab, d.h. je härter die Latices desto langsamer die Freigabe.

*CPA = Kontrollierte Percutane Application

Tabelle IV

freigegebene Menge an Clonidin in %

| Zeit in Tagen | Produkt VA 381 A | Produkt VA 381 B |
|---|---|---|
| 0,33 | 26,3 | 50,9 |
| 1 | 47,2 | 67,7 |
| 4 | 66,8 | 73,0 |
| 5 | 70,2 | 74,4 |

Filmdicke: 100 $\mu$m, Beladungsmenge 0,7 mg Clonidin/cm$^2$

B) Tabletten:

Es werden drei unterschiedliche Polyacrylattypen zusammen mit einem Wirkstoff zu Tabletten verarbeitet und deren Freigabeverhalten in Abb. II dargestellt.

Die Polymere D (Eudragit E 30 D) und E sind emulsionspolymerisierte Polyacrylate, das Polymer F (Eudragit RS 100) ein substanzpolymerisiertes Polyacrylat der Firma Röhm GmbH, Darmstadt. Die beiden Polymertypen E und F haben ungefähr die gleiche Glastemperatur (Tg~30$^\circ$C), befinden sich jedoch physikalisch in verschiedenen Formen: kugelförmiger Latex (E) bzw. als Polymerknäuel (F). Die Glastemperatur des Polyacrylats D, ebenfalls ein kugelförmiger Latex, beträgt Tg = -8$^\circ$C. Die Glastemperatur kann durch Variation des Mengenverhältnisses der das Polymer aufbauenden Monomere eingestellt werden, die Form durch die Polymerisationsbedingungen.

Beispiel 1

In einem geeigneten Gefäß werden 23,75 g des Polymeren E zusammen mit 400 ml Aceton gerührt, bis eine klare Lösung entsteht, anschließend wird die Wirkstofflösung, bestehend aus 1,25 g B-HT 933 Base in 100 ml Methanol zugegeben. Von der so erhaltenen Lösung wird das Lösungsmittel unter Schutzgasatmosphäre (N$_2$) abgedampft und der zurückbleibende wirkstoffbeladene Film mittels Trockeneis auf etwa -40$^\circ$C heruntergekühlt und zusammen mit Lactose im Mengenverhältnis 9 : 1 in einer Mühle mit rotierenden Messern unter Kühlung portionsweise gemahlen. Man erhält ein freifließendes Pulver, in dem das wirkstoffbeladene Polyacrylat logarithmisch normal verteilt zwischen 65 und 500 $\mu$m vorliegt.

Das in der oben beschriebenen Weise erhaltene Gemisch wird in einem weiteren Arbeitsschritt nach bekannten Verfahren mit üblichen Tablettierhilfsstoffen, wie z.B. Milchzucker, Maisstärke, kolloidale Kieselsäure und/oder Magnesiumstearat, zu Tabletten verpreßt, die eine 5%ige Wirkstoffbeladung, bezogen auf den Polymerträger, aufweisen.

Beispiel 2

In Analogie zu Beispiel 1 werden 95 g des Polymeren F, gelöst in 250 ml Dichlormethan und 5,0 g B-HT 933 Base in 40 ml Methanol zu einem Film verarbeitet, vermahlen und zu Tabletten verpreßt.

Beispiel 3

In Analogie zu Beispiel 1 werden 95 g des Polyacrylates E, gelost in 500 ml Aceton und 5,0 g B-HT 933 Base in 40 ml Methanol zu einem Film verarbeitet, vermahlen und zu Tabletten verpreßt.

Die in vitro-Freigabe der Tabletten wurde bei 37°C in einem USP XVII-Tester durchgeführt, wobei als Rezptormedium Wasser vom pH 7 genommen wurde. Die Analytik erfolgte mittels HPLC.

Wie aus der Abb. II ersichtlich, wird der Wirkstoff von dem substanzpolymerisierten Polyacrylat bereits nach 2 Stunden fast vollständig abgegeben, während bei einem emulsionspolymerisierten Polyacrylat in Abhängigkeit von der Glastemperatur ein gewünschtes Freigabeprofil, z.B. $t_{1h}$ 19 %; $t_{6h}$ 60 % Freigabe an Arzneistoff, eingestellt werden kann.

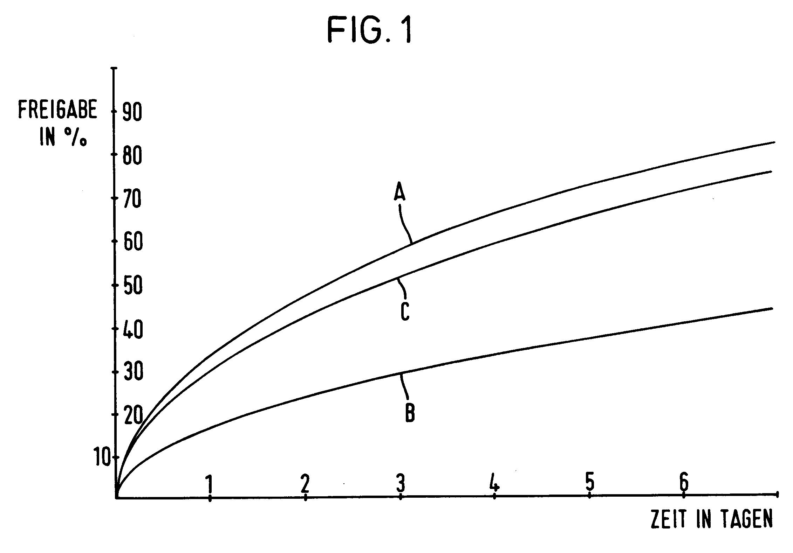

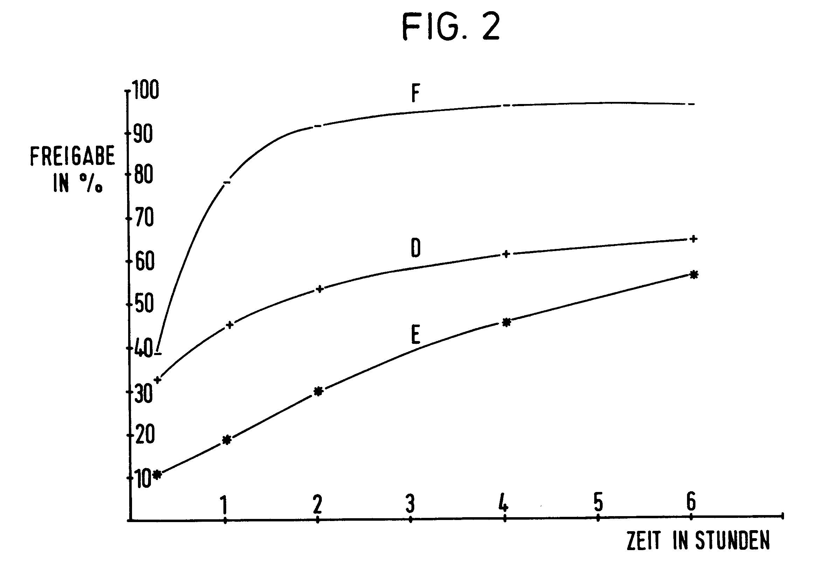

## Patentansprüche

1. Pharmazeutische Zubereitung mit variierbarer kontrollierter und verzögerter Wirkstofffreigabe, enthaltend ein Trägermaterial auf der Basis eines Emulsionspolymerisates, dadurch herstellbar, daß man ein auf eine Teilchengröße von 50 bis 500 nm polymerisiertes Emulsionspolymerisat mit einer Glastemperatur zwischen -20°C und +80°C zusammen mit dem Arzneimittelwirkstoff in einem Lösungsmittel suspendiert, wobei der Arzneimittelwirkstoff in dem Lösungsmittel auch gelöst sein kann und
   a) die wirkstoffhaltige Suspension zu einem Film gießt und nach Abdampfen des Lösungsmittels den wirkstoffhaltigen Film gegebenenfalls mit einer dampfundurchlässigen Rückschicht versieht und konfektioniert oder
   b) das Lösungsmittel abdampft, das feste wirkstoffhaltige Emulsionspolymerisat bei einer unter der Glastemperatur liegenden Temperatur auf eine Korngröße zwischen 10 und 500µm vermahlt, mit üblichen Tablettierhilfsstoffen verarbeit und zu Tabletten verpreßt oder in Kapseln füllt, wobei eine Abnahme der Teilchengröße eine Erhöhung der Freisetzungsrate, und ein Anstieg der Glastemperatur eine Verringerung der Freisetzungsrate ergeben,

2. Pharmazeutische Zubereitung nach Anspruch 1, dadurch gekennzeichnet, daß das Emulsionspolymerisat eine Glastemperatur zwischen -10 und +30°C aufweist.

3. Pharmazeutische Zubereitung im Form einer Tablette oder Kapsel nach Anspruch 1 oder 2, dadurch gekennzeichnet, daß das Trägermaterial mit dem Arzneimittelwirkstoff eine Korngröße von 10 bis 500 µm aufweist.

4. Pharmazeutische Zubereitung in Form eines Films nach Anspruch 1 oder 2, dadurch gekennzeichnet, daß der Film eine Schichtdicke zwischen 40 und 200 µm, bevorzugt 60 bis 140 µm, aufweist.

5. Pharmazeutische Zubereitung nach Anspruch 4, dadurch gekennzeichnet, daß das Polymer aus einem emulsionspolymerisierten Copolymerisat von Methyl- und/oder Ethylestern der Acryl- und Methacrylsäure besteht.

6. Pharmazeutische Zubereitung nach einem der Ansprüche 1 bis 5, dadurch gekennzeichnet, daß diese zwischen 0.1 und 30 Gew.-%, Wirkstoff enthält.

7. Pharmazeutische Zubereitung nach Anspruch 6, dadurch gekennzeichnet, daß der Wirkstoff Clonidin oder dessen pharmakologisch verträgliches Säureadditionssalz ist

8. Pharmazeutische Zubereitung in Form einer Tablette nach Anspruch 1, 2, 3, und/oder 6, dadurch gekennzeichnet, daß der Wirkstoff Clonidin oder dessen pharmakologisch verträgliches Säureadditonssalz ist. wobei Zubereitungen aus einem Emulsionspolyacrylat mit einer Teilchengröße von ca. 140 nm und mit einer Glastemperatur von ca. 30°C ausgeschlossen sind.

9. Verfahren zur Herstellung einer pharmazeutischen Zubereitung mit variierbarer kontrollierter und verzögerter Wirkstofffreigabe, enthaltend ein Trägermaterial auf der Basis eines Emulsionspolymerisates, dadurch gekennzeichnet, daß man ein auf eine Teilchengröße von 50 bis 500 nm polymerisiertes Emulsionspolymerisat mit einer Glastemperatur zwischen -20°C und +80°C zusammen mit dem Arzneimittelwirkstoff in einem Lösungsmittel suspendiert, wobei der Arzneimittelwirkstoff in dem Lösungsmittel auch gelöst sein kann und

# EP 0 209 121 B1

a) die wirkstoffhaltige Suspension zu einem Film gießt und nach Abdampfen des Lösungsmittels den wirkstoffhaltigen Film gegebenenfalls mit einer dampfundurchlässigen Rückschicht versieht und konfektioniert oder

b) das Lösungsmittel abdampft, das feste wirkstoffhaltige Emulsionspolymerisat bei einer unter der Glastemperatur liegenden Temperatur auf eine Korngröße zwischen 10 und 500$\mu$m vermahlt, mit üblichen Tablettierhilfsstoffen verarbeit und zu Tabletten verpreßt oder in Kapseln füllt, wobei eine Abnahme der Teilchengröße eine Erhöhrung der Freisetzungsrate, und ein Anstieg der Glastemperatur eine Verringerung der Freisetzungsrate ergeben,

wobei Zubereitungen aus einem Emulsionspolyacrylat mit einer Teilchengröße von ca. 140 nm und mit einer Glastemperatur von ca. 30° C ausgeschlossen sind.

## Claims

1. Pharmaceutical preparation with a variable controlled and delayed release of active substance, containing a carrier material based on an emulsion polymer, obtainable by suspending an emulsion polymer, which has been polymerised to a particle size of from 50 to 500 nm, having a glass temperature of between -20° C and +80° C, together with the pharmaceutical active substance, in a solvent, whilst the pharmaceutical active substance may also be dissolved in the solvent and

   a) pouring out the suspension which contains the active substance to form a film and, after evaporation of the solvent, optionally providing the film which contains the active substance with a vapour-impermeable backing layer and packaging said film or

   b) evaporating off the solvent, grinding the solid emulsion polymer which contains the active substance to a granule size of between 10 and 500 $\mu$m at a temperature below the glass transition temperature, processing it with conventional tablet-making excipients and compressing it to form tablets or packing it into capsules,

   any reduction in the particle size resulting in an increase in the rate of release, and any rise in the glass transition temperature resulting in a reduction in the rate of release,

   excluding preparations consisting of an emulsion polyacrylate with a particle size of about 140 nm and a glass transition temperature of about 30° C.

2. Pharmaceutical preparation as claimed in claim 1, characterized in that the emulsion polymer has a glass temperature of between -10 and +30° C.

3. Pharmaceutical preparation in the form of a tablet or capsule as claimed in claim 1 or 2, characterised in that the carrier material containing the pharmaceutical active substance has a granule size of from 10 to 500 microns.

4. Pharmaceutical preparation in the form of a film as claimed in claim 1 or 2, characterized in that the film has a layer thickness of between 40 and 200 microns, preferably from 60 to 140 microns.

5. Pharmaceutical preparation as claimed in claim 4, characterised in that the polymer consists of an emulsion-polymerised copolymer of methyl and/or ethyl esters of acrylic and methacrylic acid.

6. Pharmaceutical preparation as claimed in one of claims 1 to 5, characterized in that it contains between 0.1 and 30 wt.% of active substance.

7. Pharmaceutical preparation according to claim 6, characterised in that the active substance is clonidine or a pharmacologically acceptable acid addition salt thereof.

8. Pharmaceutical preparation in the form of a tablet according to claim 1, 2, 3, 4 and/or 6, characterised in that the active substance is clonidine or a pharmacologically acceptable acid addition salt thereof.

9. Process for preparing a pharmaceutical preparation with variable controlled and delayed release of active substance, containing a carrier material based on an emulsion polymer, characterised in that an emulsion polymer, polymerised to a particle size of from 50 to 500 nm, having a glass temperature of between -20° C and +80° C, is suspended together with the pharmaceutical active substance in a

7

EP 0 209 121 B1

solvent, whilst the pharmaceutical active substance may also be dissolved in the solvent, and

a) the suspension which contains the active substance is poured out to form a film and, after evaporation of the solvent, the film containing the active substance is optionally provided with a vapour-impermeable backing layer and packaged or

b) the solvent is evaporated off, the solid emulsion polymer which contains the active substance is ground to a granule size of between 10 and 500 $\mu$m at a temperature below the glass transition temperature, processed with conventional tablet-making excipients and compressed to form tablets or packed into capsules,

any reduction in the particle size resulting in an increase in the rate of release, and any rise in the glass transition temperature resulting in a reduction in the rate of release,

whilst preparations consisting of an emulsion polyacrylate with a particle size of about 140 nm and with a glass transition temperature of about 30 $^\circ$C are excluded.

**Revendications**

1. Préparation pharmaceutique à libération contrôlée et retardée variable de la substance active, contenant un support à base d'un polymère polymérisé en émulsion, que l'on peut préparer en mettant en suspension dans un solvant un polymère polymérisé en émulsion jusqu'à une taille de particules de 50 à 500 nm et ayant une température de transition vitreuse comprise entre -20 $^\circ$C et +80 $^\circ$C en même temps que la substance active médicamenteuse, la substance active médicamenteuse pouvant aussi être dissoute dans le solvant et

a) en coulant la suspension contenant la substance active en un film et, après évaporation du solvant, en munissant éventuellement le film contenant la substance active d'une couche dorsale imperméable à la vapeur et en le conditionnant avec celle-ci

ou

b) en évaporant le solvant, en broyant le polymère polymérisé en émulsion solide contenant la substance active jusqu'à une taille de particules comprise entre 10 et 500 $\mu$m et à une température inférieure à la température de transition vitreuse, en le traitant avec des adjuvants de compactage habituels et en le compactant en comprimés ou en l'introduisant dans des capsules,

une réduction de la taille des particules produisant une élévation de la vitesse de libération, et une montée de la température de transition vitreuse produisant une diminution de la vitesse de libération, les préparations à base de polyacrylate polymérisé en émulsion ayant une taille de particules d'environ 140 nm et une température de transition vitreuse d'environ 30 $^\circ$C étant exclues.

2. Préparation pharmaceutique selon la revendication 1, caractérisée en ce que le polymère polymérisé en émulsion présente une température de transition vitreuse comprise entre -10 et +30 $^\circ$C.

3. Préparation pharmaceutique sous forme d'un comprimé ou d'une capsule selon la revendication 1 ou 2, caractérisée en ce que le support avec la substance active médicamenteuse présente une taille de particules de 10 à 500 $\mu$m.

4. Préparation pharmaceutique sous forme d'un film selon la revendication 1 ou 2, caractérisée en ce que le film présente une épaisseur comprise entre 40 et 200 $\mu$m, de préférence entre 60 et 140 $\mu$m.

5. Préparation pharmaceutique selon la revendication 4, caractérisée en ce que le polymère consiste en un copolymère polymérisé en émulsion d'esters méthyliques et/ou éthyliques de l'acide acrylique et de l'acide méthacrylique.

6. Préparation pharmaceutique selon l'une des revendications 1 à 5, caractérisée en ce qu'elle contient entre 0,1 et 30% en poids de substance active.

7. Préparation pharmaceutique selon la revendication 6, caractérisée en ce que la substance active est la clonidine ou son sel d'addition d'acide compatible du point de vue pharmacologique.

8. Préparation pharmaceutique sous forme d'un comprimé selon la revendication 1, 2, 3 et/ou 6, caractérisée en ce que la substance active est la clonidine ou son sel d'addition d'acide compatible du point de vue pharmacologique.

8

9. Procédé de fabrication d'une préparation pharmaceutique à libération contrôlée et retardée variable de la substance active, contenant un support à base d'un polymère polymérisé en émulsion, caractérisé en ce que l'on met en suspension dans un solvant un polymère polymérisé en émulsion jusqu'à une taille de particules de 50 à 500 nm et ayant une température de transition vitreuse comprise entre -20°C et +80°C en même temps que la substance active médicamenteuse, la substance active médicamenteuse pouvant être dissoute aussi dans le solvant et

    a) on coule la suspension contenant la substance active en un film et, après évaporation du solvant, on munit éventuellement le film contenant la substance active d'une couche dorsale imperméable à la vapeur et on le conditionne avec celle-ci

    b) on évapore le solvant, on broie le polymère polymérisé en émulsion solide contenant la substance active jusqu'à une taille de particules comprise entre 10 et 500 $\mu$m et à une température inférieure à la température de transition vitreuse, on le traite avec des adjuvants de compactage habituels et on le compacte en comprimés ou on l'introduit dans des capsules,

une réduction de la taille des particules produisant une élévation de la vitesse de libération, et une montée de la température de transition vitreuse produisant une diminution de la vitesse de libération,

les préparations à base de polyacrylate polymérisé en émulsion ayant une taille de particules d'environ 140 nm et une température de transition vitreuse d'environ 30°C étant exclues.

FIG. 1

FREIGABE IN %

90
80
70
60
50
40
30
20
10

A

C

B

1    2    3    4    5    6

ZEIT IN TAGEN

EP 0 209 121 B1

FIG. 2

EP 0 209 121 B1